# EUROPEAN PATENT APPLICATION

(11) **EP 1 835 023 A1**
(43) Date of publication of application: **19.09.2007**
(21) Application number: 05811609.6
(22) Date of filing: 02.12.2005
(51) Int. Cl.: C12N 5/06, A61K 35/44, A61L 27/00, A61P 27/02

(54) **HUMAN CORNEAL ENDOTHELIAL CELL-DERIVED PRECURSOR CELL AND CELL AGGREGATE, PROCESS FOR PRODUCING THE SAME, AND METHOD FOR TRANSPLANTING PRECURSOR CELL AND CELL AGGREGATE**

(30) Priority: 09.12.2004 JP 2004356348
(71) Applicant: Amano, Shiro, Tokyo 168-0081 (JP); Yamagami, Satoru, Saitama-shi, Saitama 3380001 (JP); Mimura, Tatsuya, Tokyo 1710014 (JP)
(72) Inventor: OSAKABE, Yasuhiro, c/o HOYA CORPORATION, Tokyo 161-8525 (JP)
(74) Representative: Leidescher, Thomas
(86) International application number: PCT/JP2005/022189
(87) International publication number: WO 2006/092894

(57) **Abstract**

Providing is cellular aggregates derived from corneal endothelial cells that, when transplanted, readily adhere to the parenchyma of cornea and function in a manner equivalent to corneal endothelial cells, and a method of transplantation of the cellular aggregates. Cellular aggregates derived from corneal endothelial cells. The cellular aggregates derived from corneal endothelial cells is prepared by culturing human corneal endothelial cells in a medium containing fetal bovine serum, growth factor and glucose; and then float culturing the cells obtained in a medium containing growth factor. A method of transplantation into the anterior chamber the cellular aggregate or the cellular aggregate prepared by the above method, comprising inserting a tube into the parenchyma of cornea, introducing the cellular aggregate into the anterior chamber through the inserted tube, and causing the cellular aggregate that has been introduced to adhere to Descemet's membrane by assuming in a downward-facing position.

## Description

### [Technical Field]

The present invention relates to human corneal endothelial precursor cells derived from human corneal endothelial tissue, cellular aggregates in the form of stem cell-like cells derived from human corneal endothelial cells, and methods for manufacturing the same. The present invention further relates to methods for transplanting these precursor cells or cellular aggregates into the anterior chamber to regenerate the endothelial cell layer.

### [Background Art]

The conventional method for treating severe corneal disease is to perform a cornea transplant. However, at least in Japan, cornea donors, and thus the availability of transplants, are currently in extremely short supply. Further, there is an issue of tissue rejection with allotransplants. Thus, treatment by cornea transplant is far from being an ideal solution.

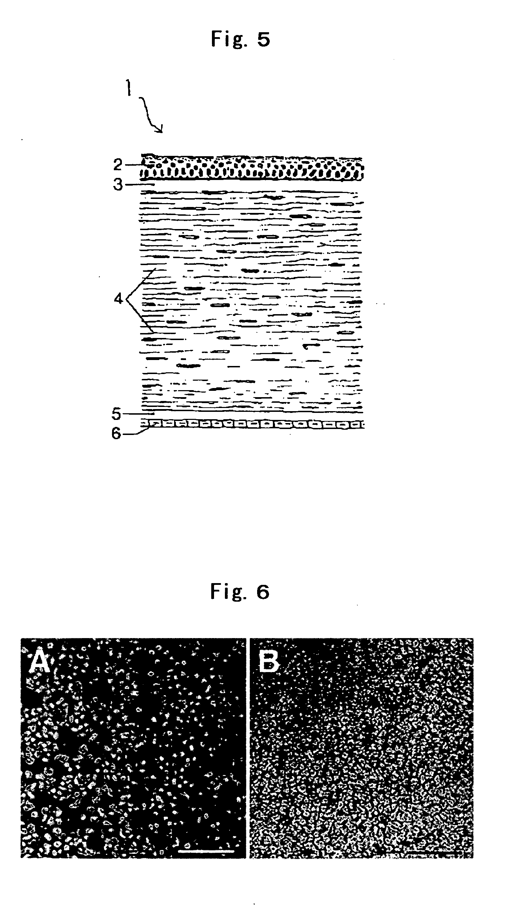

As shown in Fig. 5, the cornea 1 is comprised of a multilayered structure in the form of the anterior corneal epithelium 2, Bowman's membrane 3, the parenchyma of cornea 4, Descemet's membrane 5, and the corneal endothelium cells 6. The anterior corneal epithelium 2, which is the outermost layer, is comprised of 5 or 6 layers of nonkeratinized stratified squamous cells about 50 micrometers in thickness. The parenchyma of cornea 4 are comprised of tightly-packed, orderly collagen tissue and keratocytes, and are highly transparent. Corneal endothelial cells 6 are cells of the innermost layer. The corneal endothelial cells have a pumping function, maintaining a suitable water content within the cornea.

Attempts have been made to regenerate the cornea with corneal cells as a replacement for cornea transplants. Regeneration of the anterior corneal epithelium using stem cells is conducted as a clinical practice. Specifically, a portion of the corneal epithelium cells of a healthy corneal limbus are collected from a patient, cultured on amniotic membrane, and transplanted to promote regeneration in this method.

The method of constructing on the cornea a layer of corneal endothelial cells extracted from a patient requiring a cornea transplant is known (Japanese Unexamined Patent Publication No. 2002-78723, Patent Reference 1). Although corneal endothelial cells are transplanted to the patient's own cornea, this does not work well, as will be described further below. The stem cells of corneal endothelial cells have not yet been used to build up a layer of corneal endothelial cells.

### [Description of the Invention]

The presence of corneal endothelial stem cells has not yet been confirmed and methods of cultivating the same are as yet unknown. Accordingly, when the corneal endothelium is damaged, it is still necessary to rely on allotransplantation. However, problems such as those set forth above (insufficient donors and rejection reactions) are still unsolved. Roughly 60 percent of cases in which a cornea transplant is required are due to disease of the corneal endothelium. Accordingly, were the presence of the stem cells of corneal endothelial cells confirmed and methods of culturing them available, it would be possible to culture and transplant such cells. The availability of stem cell cultures would facilitate the growth of such cells and permit their long-term storage.

Further, as set forth above, attempts have been made to transplant corneal endothelial cells. However, the corneal endothelial cells that have been transplanted by the methods conceived thus far have been quite small, tended to move above, and been difficult to affix to the parenchyma of cornea. Thus, the transplantation of corneal endothelial cells has not reached a practical level.

Accordingly, one object of the present invention is to solve these problems by providing cells derived from corneal endothelial cells that, when transplanted, readily adhere to the parenchyma of cornea and function in a manner equivalent to corneal endothelial cells, that is, maintain a suitable water content within the cornea.

A further object of the present invention is to provide a method for regenerating the endothelial cell layer by transplanting the above cells into the anterior chamber.

To solve the above-stated problems, the present invention is as follows:
[1] Human corneal endothelial precursor cells derived from human corneal endothelial tissue.
[2] The human corneal endothelial precursor cells according to [1], in which the human corneal endothelial tissue comprises a monolayer of corneal endothelial cells and Descemet's membrane.
[3] The human corneal endothelial precursor cells according to [1] or [2], being nestin-positive and BrdU-positive.
[4] The human corneal endothelial precursor cells according to any of [1] to [3], being capable of adhering readily to the cornea when transplanted onto the cornea.
[5] The human corneal endothelial precursor cells according to any of [1] to [4], being obtainable by culturing human corneal endothelial tissue in a medium comprising growth factor and glucose under human serum, animal serum, or no-serum conditions.
[6] A method of preparation of human corneal endothelial precursor cells according to any of [1] to [4], comprising culturing human corneal endothelial tissue in a medium comprising growth factor and glucose under human serum, animal serum, or no-serum conditions.
[7] The method of preparation according to [6], wherein the culturing of the human corneal endothelial tissue is conducted in a primary culture and subcultured for 2 to 10 successive generations.
[8] The method of preparation according to [6] or [7], wherein the culturing of the human corneal endothelial tissue is conducted under conditions of 37°C and 5 to 10 percent CO₂.
[9] A cellular aggregate derived from cultured human corneal endothelial cells.
[10] The cellular aggregate according to [9], having a diameter falling within a range of 30 to 500 micrometers.
[11] The cellular aggregate according to [9] or [10], being nestin-positive, *alpha-*SMA-positive, and BrdU-positive.
[12] The cellular aggregate according to any of [9] to [11], being capable of adhering to the cornea when transplanted onto the cornea.
[13] The cellular aggregate of any of [9] to [12], wherein the number of nestin-positive cells becomes 5 percent or less when cultured for 3 to 10 days in an incubator coated with extracellular matrix.
[14] The cellular aggregate according to any of [9] to [13], being negative for *beta*-III tubulin and GFAP.
[15] The cellular aggregate according to any of [9] to [14], being capable of exhibiting a polygonal form when cultured, and permitting the formation of human corneal endothelium-like sheets from multiple cellular aggregates.
[16] The cellular aggregate according to [15], wherein said human corneal endothelium-like sheet has a transport activity equivalent to that of a normal human corneal endothelial layer.
[17] The cellular aggregate according to any of [9] to [16], being obtained by float culturing the human corneal endothelial precursor cells according to any of [1] to [5], or human corneal endothelial precursor cells prepared according to the method of any of [5] to [8].
[18] A method of preparation of cellular aggregate derived from human corneal endothelial cells comprising float culturing in a medium comprising growth factor the human corneal endothelial precursor cells according to any of [1] to [5] or human corneal endothelial precursor cells prepared by the method according to any of [6] to [8].
[19] A method of preparation of cellular aggregate derived from human corneal endothelial cells, comprising culturing human corneal endothelial cells in a medium comprising growth factor and glucose under human serum, animal serum, or no serum conditions; and then float culturing the cells obtained in a medium comprising growth factor.
[20] The method of preparation according to [19], wherein human corneal endothelial cells are cultured in a primary culture and subcultured for 2 to 10 successive generations.
[21] The method of preparation according to [19] or [20], wherein human corneal endothelial cells are cultured under conditions of 37°C and 5 to 10 percent CO₂.
[22] The method of preparation according to any of [19] to [21], wherein the concentration of glucose in the medium comprising glucose is 2.0 g/L or lower.
[23] The method of preparation according to any of [19] to [22], wherein the growth factor is one or more members selected from the group consisting of B cell growth factor (BCGF), epidermal growth factor (EGF), recombinant EGF (rEGF), and fibroblast growth factor (FGF).
[24] The method of preparation according to any of [18] to [23], wherein the growth factor is one or more members selected from the group consisting of B27, epidermal growth factor (EGF), and basic fibroblast growth factor (bFGF).
[25] A method of preparation of cellular aggregate derived from human corneal endothelial cells, comprising obtaining single cells by lysis of corneal endothelial cells with collagenase and then float culturing the cells obtained in a medium comprising growth factor.
[26] The method of preparation according to [25] wherein the growth factor is one or more members selected from the group consisting of B27, epidermal growth factor (EGF), and basic fibroblast growth factor (bFGF).
[27] The method of preparation according to any of [18] to [26], wherein the cellular aggregate has a diameter falling within a range of 30 to 500 micrometers.
[28] A human corneal endothelium-like sheet, comprised of human corneal endothelium-like cells derived from the cellular aggregate according to any of [8] to [17] or a cellular aggregate prepared by the method according to any of [18] to [27], wherein the endothelium-like cells exhibit a polygonal form.
[29] The sheet according to [28], wherein the polygonal form is a hexagon.
[30] The sheet according to [28] or [29], wherein the mean cell density is 2,000 cells/mm² or greater.
[31] The sheet according to any of [28] to [30], having transport activity.
[32] The sheet according to [31], wherein the transport activity is equivalent to the transport activity of a normal human corneal endothelial layer.
[33] The sheet according to any of [28] to [32], wherein the human corneal endothelium-like sheet is in the form adhered to a biodegradable support.
[34] The sheet according to [33], wherein the support is one or more member selected from the group consisting of amniotic membrane, collagen membrane, cellulose membrane, and gelatin film.
[35] A method of transplantation of human corneal endothelial precursor cells according to any of [1] to [5] or human corneal endothelial precursor cells prepared by the method according to any of [6] to [8] into the anterior chamber, comprising inserting a tube into the parenchyma of cornea, introducing the precursor cells into the anterior chamber through the inserted tube, and causing the human corneal endothelial precursor cells that have been introduced to adhere to Descemet's membrane.
[36] A method for transplanting into the anterior chamber the cellular aggregate according to any of [9] to [17] or the cellular aggregate prepared by the method according to any of [18] to [27], comprising inserting a tube into the parenchyma of cornea, introducing the cellular aggregate into the anterior chamber through the inserted tube, and causing the cellular aggregate that has been introduced to adhere to Descemet's membrane.
[37] The method according to [35] or [36] wherein the quantity of human corneal endothelial precursor cells introduced during single transplantation falls within a range of from 5,000 to 50,000 cells, or the quantity of cellular aggregate introduced falls within a range of 30 to 200 cellular aggregates.
[38] The method according to any of [35] to [37] wherein the human corneal endothelial precursor cells or cellular aggregate is transplanted into the anterior chamber in the form of a mixture with a biodegradable support material.
[39] The method according to [38] wherein the support material is a collagen sponge or gelatin microparticles.
[40] A method of transplantation of the human corneal endothelium-like sheet according to any of [28] to [34] into the anterior chamber, comprising inserting a tube into the parenchyma of cornea, introducing the human corneal endothelium-like sheet into the anterior chamber through the tube that has been inserted, and causing the corneal endothelium-like sheet that has been introduced to adhere to Descemet's membrane.
[41] The method according to any of [35] to [40] wherein the tube employed to introduce the human corneal endothelium precursor cells, cellular aggregate, or human corneal endothelium-like sheet is inserted into the parenchyma of cornea to a depth exceeding the thickness of the parenchyma of cornea.
[42] The method according to any of [35] to [41], wherein the introduction of the human corneal endothelium precursor cells, cellular aggregate, or human corneal endothelium-like sheet into the anterior chamber is conduced after air is introduced into the anterior chamber, and a downward-facing position is assumed.
[43] The method according to [42] wherein once the human corneal endothelium precursor cells, cellular aggregate, or human corneal endothelium-like sheet has been introduced into the anterior chamber, the downward-facing state is maintained for a prescribed period.
[44] The method according to any of [35] to [43] wherein a solution containing human corneal endothelium precursor cells or cellular aggregate, or a corneal endothelium-like sheet, is introduced into the anterior chamber with an injector.
[45] The method according to any of [35] to [44], being used to treat a disorder that causes corneal edema by reducing the number of corneal endothelial cells.
[46] The method of [45], wherein the disorder is vesicular keratopathy, congenital hereditary endothelial corneal dystrophy, Fuchs' endothelial corneal dystrophy, guttate cornea, posterior polymorphic corneal dystrophy, iridocorneal endothelial syndrome, or a failed corneal transplant.
[47] The method of [46], wherein the vesicular keratopathy is vesicular keratopathy following eye surgery, a laser iridectomy, uveitis, or an external injury.

### Advantages of the Invention

The present invention provides human corneal endothelial precursor cells derived from human corneal endothelium tissue. The present invention further provides a cellular aggregate in the form of stem cell-like cells derived from corneal endothelial cells. When the precursor cells or cellular aggregate is transplanted onto the parenchyma of cornea (Descemet's membrane, which is the base membrane of corneal endothelial cells), it adheres readily, forming a membrane functioning as a corneal endothelium. Thus, the corneal endothelium can be regenerated without a corneal transplant.

### [Best Mode of Implementing the Invention]

### [Human corneal endothelial precursor cells derived from human corneal endothelial tissue]

The present invention relates to human corneal endothelial precursor cells derived from human corneal endothelial tissue. The term "derived from human corneal endothelial tissue" means obtained by culturing using human corneal endothelial tissue as the starting material. Human corneal endothelial tissue includes the monolayer of human corneal endothelial cells and Descemet's membrane.

The human corneal endothelial precursor cells of the present invention are desirably positive for nestin and BrdU (bromodeoxyuridine). The fact that human corneal endothelial precursor cells derived from human corneal tissue are nestin-positive means that their development has halted in an undifferentiated state in which they are multifunctional and capable of proliferation.
The fact that they are BrdU-positive means that the cells have growth activity.

The human corneal endothelial precursor cells of the present invention are obtained by culturing human corneal endothelial tissue in a medium containing growth factor and glucose under human serum, animal serum, or no-serum conditions. More specifically, tissue containing human corneal endothelial cells can be cultured in medium containing fetal bovine serum, growth factor, and glucose.

A common medium such as M199, DMEM, HamF12, DMEM/F12, TC199, or OptiMEM can be employed. Instead of fetal bovine serum, a no-serum medium (such as ACF or HSM medium) can be employed; the ability to proliferate during culturing can be enhanced in a medium to which a 1 to 10 percent concentration of human serum has been added.

The glucose concentration in the glucose-containing medium is lower than the glucose concentration in common glucose-containing media. A concentration of 2.0 g/L or less is desirable. Specifically, a range of 0.1 to 2.0 g/L, preferably a range of 0.1 to 1.0 g/L, is employed.

The concentration of fetal bovine serum (FBS) is 10 to 15 percent, for example.

Examples of growth factor suitable for use are: B cell growth factor (BCFG), epidermis growth factor (EGF), recombinant EGF (rEGF), and fibroblast growth factor (FGF). These may be employed singly, or in suitable combinations of two or more, in the medium. The concentration of these growth factors is 1 to 5 ng/mL, preferably 1 to 2 ng/mL.

The culture temperature is 35 to 38°C, preferably 37°C. Culturing is conducted in an incubator at 90 to 100 percent humidity (preferably 100 percent humidity) and 5 to 10 percent CO₂ (preferably 10 percent CO₂). The cells are subcultured when they reach a dense stage (about 7 to 10 days following the saturation state). Subculturing is suitably conducted while observing the state of growth of the cells; the subculturing of about 2 to 10 generations suffices.

### [Cellular aggregate derived from human corneal endothelial cells]

The present invention relates to cellular aggregate derived from human corneal endothelial cells.
The term "derived from human corneal endothelial cells" means the obtaining of human corneal endothelial cells by separating Descemet's membrane, comprising a corneal endothelial monolayer, from human corneal tissue and conducting enzymatic treatment to separate the endothelial cells. The cellular aggregate exhibits a spherical shape, and may also be referred to as "spherical cellular masses" or "spheroids." Here, the term "spherical" is used to mean both truly spherical shapes and shapes that are approximately spherical. The cross section of the sphere in the present invention may consist of concentric circles, concentric polygons, oval circles (such as ellipses and rugby ball shapes), and elliptical polygons (such as hexagons and above).

The cellular aggregate desirably has a diameter falling within a range of 30 to 500 micrometers, preferably a range of 30 to 300 micrometers, and still more preferably, a range of 30 to 100 micrometers. As is set forth further below, the cellular aggregate can be formed by float culturing cells in an undifferentiated state, such as corneal epithelial precursor cells, so that cells with homogeneous properties, that is, cells having undifferentiated capabilities, gather together into an aggregate. This formation of an aggregate facilitates the subsequent growth and development of the cells.

The cellular aggregate of the present invention is desirably nestin-positive, positive for *alpha-SMA* (alpha-smooth muscle actin), and BrdU-positive. Being nestin-positive means that the cells constituting the cellular aggregate have remained in an undifferentiated state that is multifunctional and capable of proliferation. Being *alpha*-SMA positive means that cells of the muscle fibroblast system, derived from the mesenchymal system, are contained. Being BrdU-positive means that the cells have growth activity.

A cellular aggregate of the present invention in which 5 percent or less of the cells are nestin-positive is obtained by culturing the cells in an incubator (for 3 to 10 days) that has been coated with an extracellular matrix.
At day 6 to 8 from the start of float culturing, corneal endothelial precursor cells are inoculated onto a cover glass that has been coated with extracellular matrix. They are then cultured for another 7 days in a medium to which have been added about 1 percent of FBS, about 20 ng/mL of EGF, and about 20 ng/mL of bFGF. About 10 micrograms/mL of fibronectin can be employed in the extracellular matrix.

The reason that 5 percent or less of the cells are nestin-positive is as follows. It is widely known that being positive for nestin is an indicator of undifferentiated stem cell-like cells, that is, an indicator that cells have remained in an undifferentiated state in which they are multifunctional and capable of proliferation; this is not limited to human corneal endothelial precursor cells derived from human corneal tissue. By contact culturing cellular aggregate containing numerous corneal endothelial precursor cells under the above culture conditions to cause differentiation into human corneal endothelium-like cells, 5 percent or less of the cells will be nestin-positive.

In the present invention, the cellular aggregate obtained by culturing is negative for *beta*-III tubulin and glial fibrillary acidic protein (GFAP). Being negative for *beta*-III tubulin indicates that no cells of the neuron cell series of nervous system cells are contained. Being negative for GFAP means that no cells of the glial cell series are contained. In other words, this means that undifferentiated stem cell-like corneal endothelial precursor cells belonging to the corneal endothelial cell series are the primary constituents.

The cellular aggregate of the present invention is an aggregate of stem cell-like cells. Following transplantation onto the cornea, they are capable of affixing themselves, and following fixation, perform functions equivalent to those of corneal endothelial cells; that is, they function to maintain a suitable moisture content within the cornea. This point will be further described through embodiments.

The cellular aggregate of the present invention exhibits a polygonal form when cultured, and can form a human corneal endothelium-like sheet comprised of multiple cells. This human corneal endothelium-like sheet has transport activity equivalent to a normal human corneal endothelial layer.

The method for preparing a cellular aggregate derived from human corneal endothelial cells of the present invention will be described below.
The cellular aggregate of the present invention can be prepared by float culturing the above precursor cells derived from human corneal endothelial tissue in a medium containing growth factor.
Although human corneal endothelial cells can be directly float cultured in a medium containing growth factor, cellular aggregate production efficiency is sometimes poor.

It is also possible to prepare cellular aggregate without culturing. However, the float culturing of precursor cells is desirable because it yields numerous cellular aggregates. When preparing cellular aggregate without using precursor cells, corneal endothelial cells are dissolved in collagenase (0.02 percent, for example) to obtain single cells. The single cells can then be float cultured in a medium containing growth factor in the same manner as set forth above. The dissolution in collagenase can be conducted overnight in a CO₂ incubator at 37°C, for example.

The above float culturing of precursor cells is conducted in a culture solution containing growth factor. The growth factor employed can be one or more members selected from the group consisting of B27, epidermal growth factor (EGF), and basic fibroblast growth factor (bFGF), for example. The concentration of growth factor in the culture solution is 10 to 60 ng/mL. For B27 and epidermal growth factor (EGF), this concentration is desirably about 20 nm/mL, and for basic fibroblast growth factor (bFGF), desirably about 40 ng/mL.

B27 is a serum replacement additive originally developed for the long-term stabilization and culturing of neurons of the hippocampus and other central nervous systems. Since cells can only be maintained in an undifferentiated state in the absence of serum, these cells must be cultured in the absence of serum. Thus, the serum replacement B27 is required.

To prevent reaggregation of cells in the above-described culture solution, methyl cellulose gel matrix can be incorporated. However, the incorporation of methyl cellulose gel matrix sometimes reduces the sphere recovery rate. Thus, this point must be taken into account when determining whether or not to employ methyl cellulose gel matrix, and what quantity to employ. When employed, methyl cellulose gel matrix, is desirably utilized in a quantity falling within a range of 4.0 to 15.0 g/L, preferably a range of 6.0 to 10.0 g/L, for example.

A cellular aggregate derived from human corneal endothelial cells can be obtained by float culturing the precursor cells of human corneal endothelial cells in a culture solution containing growth factor. The cellular aggregate will grow based on the period of float culturing; for example, it may reach a diameter falling within a range of 30 to 500 micrometers.

### [Human corneal endothelium-like sheet]

The present invention covers human corneal endothelium-like sheet comprised of endothelium-like cells derived from the above-described cellular aggregate of the present invention or cellular aggregate prepared by the method of the present invention, in which the endothelium-like cells exhibit a polygonal shape.

The human corneal endothelium-like sheet of the present invention is prepared by further culturing the cellular aggregate of the present invention. The cellular aggregate is further cultured so that the endothelium-like cells in the cellular aggregate exhibit a polygonal form. The polygonal form may be hexagonal, for example. In some cases, it will be a regular hexagon, and in other cases, the six angles or the six sides of the hexagonal may not be equal. There are also cases where the six sides of the hexagon may be curved (for example, bowed) instead of being straight lines.

The density of the cells constituting the human corneal epithelium-like sheet of the present invention is desirably 2,000 cells/mm² or greater, preferably falling within a range of 2,500 to 4,000 cells/mm², and more preferably, falling within a range of 3,000 to 4,000 cells/mm². When the cell density is excessively low, the pumping function of the transplanted human corneal endothelium-like sheet is inadequate relative to the original pumping function of corneal endothelium tissue. This results in inadequate discharge of water, causing the cornea to remain thick and presenting the possibility of the cornea continuing in a nontransparent, clouded state. Even when transparency is achieved, this transparency only lasts for a short period, presenting the problems of a short-lived cure and subsequent recidivism. When the cell density is excessively high, the sheet tends to become difficult to prepare.

The human corneal endothelium-like sheet of the present invention has transport activity. In particular, this transport activity is equivalent to that of a normal human corneal endothelium layer. The phrase " this transport activity is equivalent to that of a normal human corneal endothelium layer" means that when the sheet is transplanted and has affixed, it functions as a substitute for the human corneal endothelium.

The human corneal endothelium-like sheet is prepared by culturing the cellular aggregate or precursor cells of the present invention, preferably the cellular aggregate, in DMEM containing 10 percent FBS, for example, on a suitable support, preferably a support sheet, for example. More specifically, the human corneal endothelium-like sheet can be prepared as follows. Spherical cell masses of the precursor cells of the present invention are separated with 0.05 percent trypsin/0.02 percent EDTA to obtain single cells. The single cells obtained are inoculated onto a suitable support to a density of 3,000 cells/mm² and cultured for 2 to 4 days in a culture solution such as DMEM containing 10 to 15 percent FBS, under conditions of 37°C and 5 percent CO₂. Alternatively, the precursor cell or spherical cell masses themselves can be inoculated onto a suitable support to a density of 5 to 20 masses/mm² and cultured for 5 to 10 days in a culture solution containing 10 to 15 percent FBS under conditions of 37°C and 5 percent CO₂.

The human corneal endothelium-like sheet formed into the shape of the support is comprised of a cellular monolayer in which endothelium-like cells are adjacent to one another. The thickness falls within a range of about 5 to 20 micrometers, for example.

### [The transplantation method]

The present invention includes a method for transplanting precursor cells, a cellular aggregate, or a human corneal endothelium-like sheet prepared by the preparation method of the present invention into the anterior chamber. In this method, a tube is inserted into the parenchyma of cornea; the precursor cells, cellular aggregate, or human corneal endothelium-like sheet is introduced into the anterior chamber through the tube that has been inserted; and the precursor cells, cellular aggregate, or human corneal endothelium-like sheet that has been introduced is adhered to Descemet's membrane. An example of the transplantation of cellular aggregate will be described below.

More specifically, a solution containing cellular aggregate can be introduced into the anterior chamber with an injector. The tube used to introduce the cellular aggregate is desirably inserted into the parenchyma of cornea to a depth exceeding the thickness of the parenchyma of cornea. That is, during insertion into the eye, the syringe is caused to penetrate the cornea as close as possible to the horizontal, desirably forming as long a tunnel as possible into the parenchyma of cornea. This makes it possible to prevent leakage of water in the anterior chamber following transplantation. The medium of the solution containing the cellular aggregate may be phosphate buffer solution or BBS Plus (Alcon), for example.

Adhesion of the cellular aggregate to Descemet's membrane can be achieved even when the cellular aggregate is introduced into the anterior chamber without introducing air, but the cellular aggregate is desirably introduced into the anterior chamber following the introduction of air into the anterior chamber. By introducing air into the anterior chamber and positioning the cornea downward prior to introducing the cellular aggregate into the anterior chamber, it is possible to more efficiently cause the cellular aggregate that has been introduced to adhere to Descemet's membrane.

The precursor cells or cellular aggregate can also be introduced into the anterior chamber as a mixture with a biodegradable support material or in the form adhered to a biodegradable support.

Any biopolymer that is biodegradable may be suitably employed as the support material. However collagen (type I or IV) sponge microparticles, gelatin microparticles, and the like make particularly good culture matrixes for corneal endothelial precursor cells. Alternatively, MPC polymer microparticles comprised of 2-methacryloylyloxyethylphosphoryl choline (MPC) or the like may be employed. Any support material to which the endothelial precursor cells or suspended cells of the present invention adhere well may be employed so long as it does not exhibit cell toxicity. Transplantation along with a support material increases the cell retention time in the anterior chamber and promotes adhesion of the cells to Descemet's membrane. As a result, growth differentiation of the transplanted corneal endothelial precursor cells into Descemet membrane forms is promoted.

Any of the culture matrixes of corneal endothelial cells that are suitable for use, such as collagen thin films (types I and IV), gelatin thin films, amniotic membrane, and cellulose thin films, may be employed as supports. These may also be processed into thicknesses of 5 to 10 micrometers for use. Thin films made of materials that can be employed as artificial culture matrixes, such as 2-methacryloylyloxyethylphosphoryl choline (MPC), may be employed so long as they afford bio compativility. Round supports measuring 0.5 to 10 mm in diameter and square supports measuring 0.5 mm to 10 mm on a side may be employed irrespective of shape.

The cellular sheet formed on the support may be processed into a thin film by a biochemical method, such as the use of an enzyme, or a physical method, such as the use of a laser, and transplanted.

Following introduction of the cellular aggregate into the anterior chamber, it is desirable for the patient receiving the transplant to remain in a downward-facing position for a prescribed period. The term "prescribed period" means 6 to 24 hours. Doing this enhances adhesion to Descemet's membrane by the cellular aggregate that has been introduced.

The quantity of cellular aggregate introduced in a single transplant can be suitably determined based on the condition of the patient requiring the transplant. By way of example, a quantity falling within a range of 30 to 200 cellular aggregates may be introduced.

According to the method for transplanting the cellular aggregate of the present invention into the anterior chamber, it is possible to cause the cellular aggregates that are introduced into the anterior chamber to adhere to Descemet's membrane, regenerating the corneal endothelium layer on Descemet's membrane. The same holds true for precursor cells and human corneal endothelium-like sheet.

The methods of the present invention for transplanting precursor cells, cellular aggregate, and human corneal endothelium-like sheet into the anterior chamber are effective for all disorders that cause corneal edema by reducing the number of corneal endothelial cells. More specific examples of such disorders are: vesicular keratopathy (such as vesicular keratopathy following eye surgery, laser iridectomy, uveitis, or an external injury), congenital hereditary endothelial corneal dystrophy, Fuchs' endothelial corneal dystrophy, guttate cornea, posterior polymorphic corneal dystrophy, iridocorneal endothelial syndrome, and failed corneal transplants.

### [Embodiments]

The present invention is further described below through embodiments.

### Embodiment 1

### [Preparation of the precursor cells of human corneal endothelial cells]

Corneal endothelial cells were collected from the Descemet membrane of strong sections of cornea following a full cornea transplant and primarily cultured. The cells were cultured in DMEM supplemented with 15 percent FBS at 100 percent humidity in the presence of 5 percent CO₂. Cultured endothelial cells were separated from cell plates when the cell density reached saturation and subcultured for three generations, yielding precursor cells.

### Embodiment 2

### [Preparation of cellular aggregate from cultured corneal endothelial cells]

The neurosphere method was employed as the cell culture technique. A culture solution to which 8 g/mL of methyl cellulose gel matrix had been added to prevent cellular reaggregation was employed. The base medium employed was DMEM /F12 (1:1, Sigma) to which were added 20 ng/mL of B27 (Invitrogen, San Diego, CA), 20 ng/mL of epidermal growth factor (EGF, Sigma), and 40 ng/mL of basic fibroblast growth factor (bFGF, Sigma). Stock cells (precursor cells) were inoculated to 50 cells/microliter (50,000 cells per well) into 24-well culture plates without coatings. Cell reaggregation did not occur under these conditions. The CO₂ concentration was 5 percent.

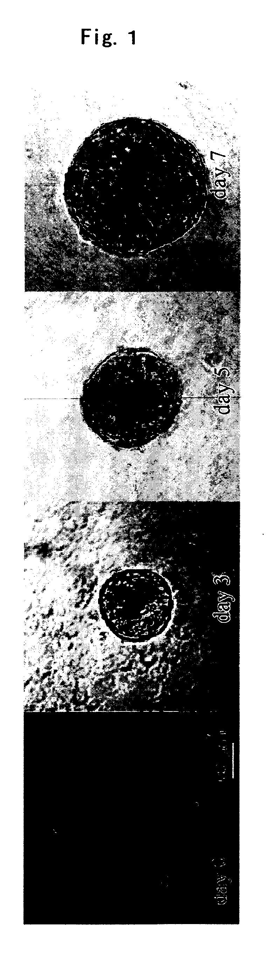

After 7 days of culturing, the cells had grown and formed cellular aggregates. Fig. 1 shows the status of the cellular aggregates at the outset of culturing, on the first day (day 0), and on days 3, 5, and 7. The diameter of the cellular aggregates on day 7 of culturing was about 400 micrometers.

BrdU staining of the cellular aggregates was examined on day 7. In BrdU staining, mouse anti-5-bromo-2'-deoxyuridine(BrdU) /fluorescent mAb was employed as primary antibody and fluorescence-labeled mouse IgG and fluorescence-labeled anticode IgG were employed as secondary antibodies, with the fluorescence being observed by fluorescence microscopy. As a result, the cellular aggregates were found to be positive for BrdU staining on day 7.

Staining of cellular aggregates was also examined for *alpha*-SMA, *beta-*III tubulin, and glial fibrillary acidic protein (GFAP) on day 7. For nestin, mouse anti-nestin mAb; for *alpha*-SMA, mouse anti-*alpha*-smooth muscle actin mAb; for *beta*-III tubulin, rabbit anti-*beta*-III tubulin polyclonal antibody; and for GFAP, rabbit anti-GFAP pAb was employed as the primary antibody. Fluorescence-labeled anti-mouse IgG and fluorescence-labeled anti-goat IgG were employed as secondary antibodies, with fluorescence being observed by fluorescence microscopy. As a result, the cellular aggregates were positive for nestin and *alpha*-SMA and negative for beta-III tubulin and GFAP on day 7.

### Embodiment 3

### [Transplantation of stem cell-like cells from cultured corneal endothelial cells]

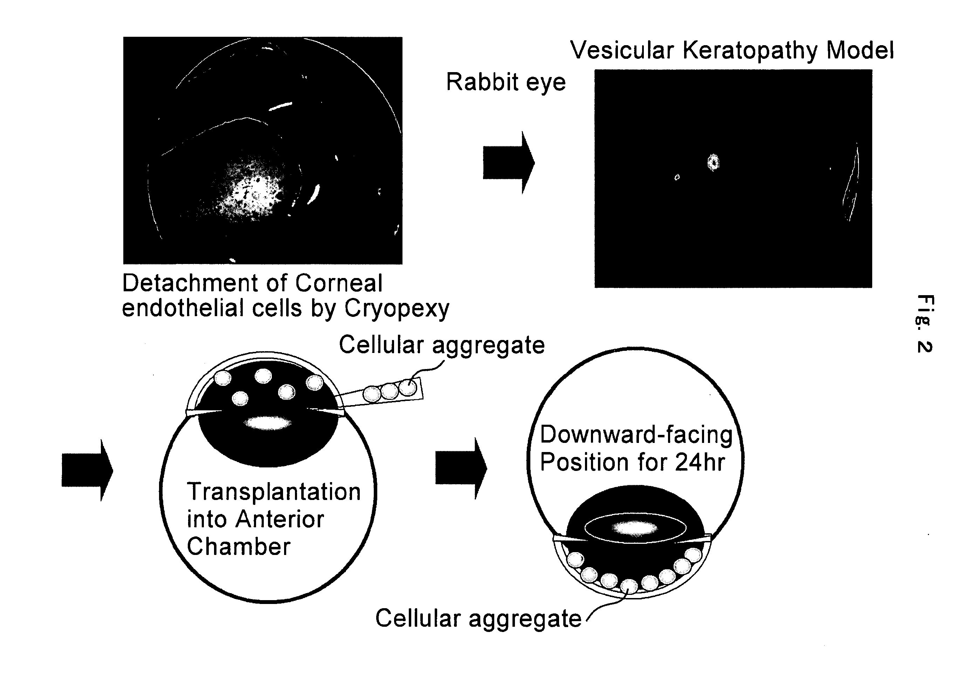

The cellular aggregate obtained in Embodiment 2 was transplanted with an injector into the anterior chamber of a rabbit eye in which corneal endothelial cells had been detached by cryopexy. That is, all of the cellular aggregates in the 30 to 500 micrometer range that had been obtained by the above-described method were collected in uncoated 60 millimeter cell plates containing PBS so that the cells did not adhere. The cellular aggregates were then centrifuged and, without replacing the solution, those cells that were spherical under a stereoscope were recovered. The cellular aggregates were then washed several times with PBS by replacement of the PBS. A 300 microliter quantity of solution containing 30 to 200 cellular aggregates was introduced into the anterior chamber of each eye. A 27 or 30 G gauge was employed in this process. A downward-facing position was maintained for 24 hours following the transplant to immobilize the cellular aggregates on the parenchyma of cornea (see Fig. 2).

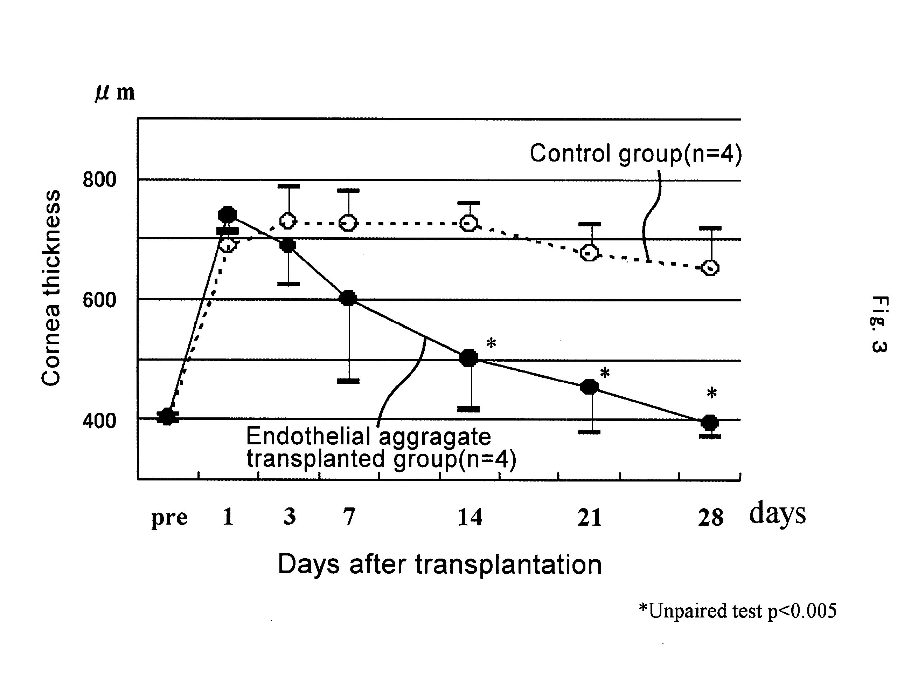

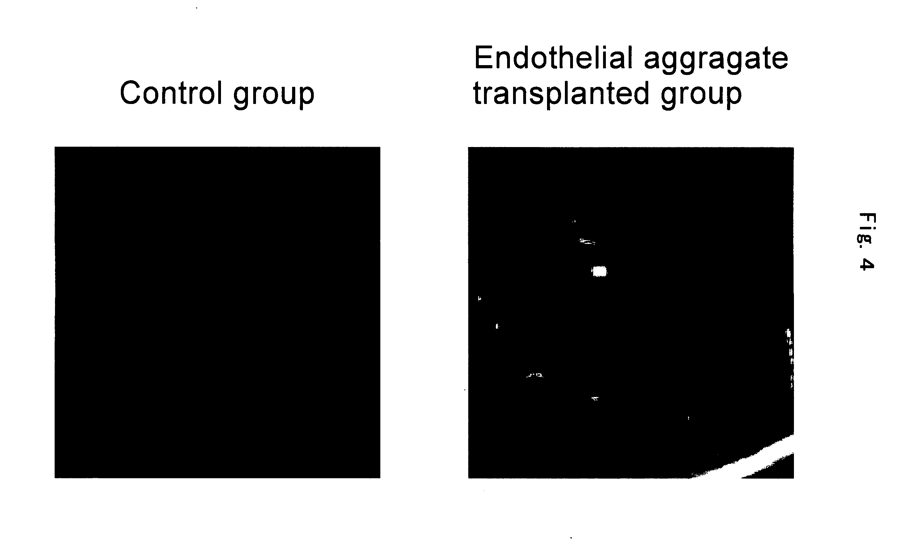

The thickness of the cornea was measured following the transplant and compared to that of a control group in which corneal endothelial cells had been detached but which had not received transplants. The results are given in Fig. 3. While the thickness of the cornea in the control group did not change greatly, the thickness of the cornea in the group that received the cellular aggregate transplant was approximately identical to that prior to the transplant after 28 days, and the transplanted cellular aggregates functioned similarly to corneal endothelial cells. That is, they functioned to maintain a suitable water content within the cornea. Fig. 4 shows photographs of the transplant cases and control cases 28 days after transplantation. In the transplant cases, corneal transparency was confirmed; the figures shows that the transplanted cellular aggregates differentiated to form a corneal endothelium-like cell layer functioning similarly to corneal endothelium cells. By contrast, the cornea was slightly opaque in the control cases.

### Embodiment 4

### [Preparation of human corneal endothelium-like sheet]

Cellular aggregates prepared from human corneal endothelial cells that had been subcultured for 5 generations were used to obtain human corneal endothelial cell sheets. Specifically, the process was conducted as follows. Cultured corneal endothelial cells were inoculated onto a suitable amniotic membrane to a density of 3,000 cells/mm² and cultured for two days in DMEM supplemented with 10 percent FBS under conditions of 37°C and 5 percent CO₂ to obtain cultured endothelial groups. Cultured corneal endothelial cells were float cultured for 7 days by the method for obtaining precursor cells or cellular aggregates of the present invention and then dispersed with 0.05 percent trypsin/0.02 percent EDTA. The single cells obtained were inoculated onto amniotic membranes to a density of 3,000 cells/mm² and cultured for 2 days in DMEM containing 10 percent FBS under conditions of 37°C and 5 percent CO₂ to obtain endothelial groups derived from cellular aggregates.

The cellular aggregate-derived endothelial group sheets that were obtained are shown immediately following inoculation in Fig. 6A and on day 2 following inoculation in Fig. 6B. The endothelial cell densities of the sheets obtained were measured. In contrast to a mean cell density of 2,819 t 124 cells/mm² achieved with the cultured endothelial groups, a mean cell density of 3,819 ±192 was achieved with the cellular aggregate-derived endothelial groups. Thus, a significantly higher endothelial cell density was achieved with the cellular aggregate-derived endothelial groups (p = 0.00051, unpaired t-test).

Based on these results, it was found that once cellular aggregates had been prepared, endothelium-like cells derived from the cellular aggregates could be employed to prepare cultured corneal endothelial cell sheets having greater cell density and a high ratio of hexagonal cells in the manner of endothelial cells.

### Embodiment 5

### [The ability to proliferate and the form of the cells]

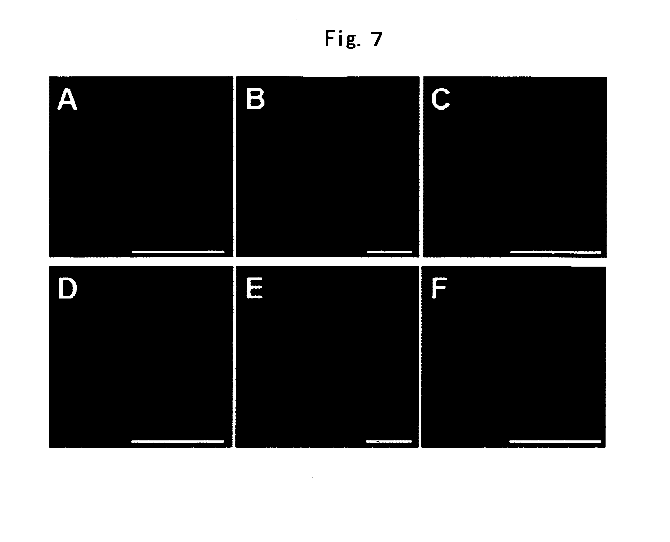

The ability to proliferate, presence of corneal epithelial cells, and cellular form were examined for the cells derived from cultured human corneal endothelial cells and the cells of the cellular aggregate obtained in Embodiment 2. The results are given in Fig. 7.

Cellular sheets were immobilized for 10 minutes with 4 percent paraformaldehyde. The cells being examined for proliferation ability were reacted overnight with 10 microM/mL of bromodeoxyuridine (BrdU) prior to immobilization. To prevent nonspecific reaction with antibodies, these cell sheets were reacted for 30 minutes with PBS containing 3 percent serum and 0.3 percent Triton X-100, after which the cell sheets that had been reacted with BrdU were reacted for 2 hours with FITC-labeled anti-BrdU antibody (1:100; Roche Diagnostics). The other cell sheets were reacted for two hours with mouse anti-cytokeratin 3 antibody (1:10,000, AE-5, Progen Biotechnik GMBH) and then labeled with FITC-labeled anti-mouse antibody (AlexaFluor 488, 1:2,000; Molecular Probes) to examine the presence of corneal epithelial cells, or reacted for two hours with rabbit anti-human-ZO-1 antibody (1:400; Zymed) and then labeled with PE-labeled anti-rabbit antibody (AlexaFluor 594, 1:400; Molecular Probes) to examine cellular form, with observation being conducted by fluorescence microscopy.

A, B, and C denote endothelial cell groups prepared from cellular aggregates, and D, E, and F denote cultured human endothelial cell groups, all derived from the same cells. BrdU staining revealed that BrdU-positive cells, indicating the ability of the cells to proliferate, were more numerous among endothelial cell group (A) prepared from spherical cells than in cultured human endothelial cell group (D). No cells positive for cytokeratin 3, found in corneal epithelial cells, was detected in either group (B, E). Endothelial cell group C, prepared from cellular aggregates, presented sharply-defined hexagonal cells, while cultured human endothelial cell group F presented numerous amorphous cell forms.

The transport activity of the cultured corneal endothelial cell sheet prepared from group B was examined by measuring the changes over time in short-circuit current and potential difference. The short-circuit current and potential difference values after 1, 5, and 10 minutes were estimated to fall within a range of about 80 to 100 percent of the values of a normal human corneal endothelial layer. This indicates that the cultured corneal endothelial cell sheets prepared from group B had suitable transport activity.

### [Industrial Applicability]

The methods of the present invention for preparing human corneal endothelial cell-derived precursor cells and cellular aggregates provide transplantable and fixable human corneal endothelial stem-cell precursor cells and cellular aggregates. Human corneal endothelium-like sheets can also be obtained from the human corneal endothelial stem-cell precursor cells or cellular aggregates, these sheets also being transplantable and fixable. The present invention is extremely useful in the field of human corneal epithelium regenerative treatment.

### [Brief Description of the Drawings]

[Fig. 1] Shows the status of spherical cells at the start of culturing, on the initial day (day 0), and at days 3, 5, and 7.
[Fig. 2] A drawing descriptive of the method of transplanting and immobilizing stem cell-like cells on the parenchyma of cornea.
[Fig. 3] The results of measurement of changes in the thickness of the cornea following the transplantation of stem cell-like cells.
[Fig. 4] Photographs of a transplantation case and control case 28 days after transplantation.
[Fig. 5] A descriptive sectional view of the cornea.
[Fig. 6] A photograph of a human corneal endothelium-like sheet derived from the cellular aggregate prepared in Embodiment 4.
[Fig. 7] Test results (fluorescent images) of the ability to proliferate of the cells in Embodiment 5.

## Claims

1. Human corneal endothelial precursor cells derived from human corneal endothelial tissue.

2. The human corneal endothelial precursor cells according to claim 1, wherein the human corneal endothelial tissue comprises a monolayer of corneal endothelial cells and Descemet's membrane.

3. The human corneal endothelial precursor cells according to claim 1 or 2, being nestin-positive and BrdU-positive.

4. The human corneal endothelial precursor cells according to any of claims 1 to 3, being capable of adhering readily to the cornea when transplanted onto the cornea.

5. The human corneal endothelial precursor cells according to any of claims 1 to 4, being obtainable by culturing human corneal endothelial tissue in a medium comprising growth factor and glucose under human serum, animal serum, or no-serum conditions.

6. A method of preparation of human corneal endothelial precursor cells according to any of claims 1 to 4, comprising culturing human corneal endothelial tissue in a medium comprising growth factor and glucose under human serum, animal serum, or no-serum conditions.

7. The method of preparation according to claim 6, wherein the culturing of the human corneal endothelial tissue is conducted in an primary culture and subcultured for 2 to 10 successive generations.

8. The method of preparation according to claim 6 or 7, wherein the culturing of the human corneal endothelial tissue is conducted under conditions of 37°C and 5 to 10 percent CO₂.

9. A cellular aggregate derived from cultured human corneal endothelial cells.

10. The cellular aggregate according to claim 9, having a diameter falling within a range of 30 to 500 micrometers.

11. The cellular aggregate according to claim 9 or 10, being nestin-positive, *alpha*-SMA-positive, and BrdU-positive.

12. The cellular aggregate according to any of claims 9 to 11, being capable of adhering to the cornea when transplanted onto the cornea.

13. The cellular aggregate of any of claims 9 to 12, wherein the number of nestin-positive cells becomes 5 percent or less when cultured for 3 to 10 days in an incubator coated with extracellular matrix.

14. The cellular aggregate according to any of claims 9 to 13, being negative for *beta*-III tubulin and GFAP.

15. The cellular aggregate according to any of claims 9 to 14, being capable of exhibiting a polygonal form when cultured, and permitting the formation of human corneal endothelium-like sheets from multiple cellular aggregates.

16. The cellular aggregate according to claim 15, wherein said human corneal endothelium-like sheet has a transport activity equivalent to that of a normal human corneal endothelial layer.

17. The cellular aggregate according to any of claims 9 to 16, being obtained by float culturing the human corneal endothelial precursor cells according to any of claims 1 to 5, or human corneal endothelial precursor cells prepared according to the method of any of claims 5 to 8.

18. A method of preparation of cellular aggregate derived from human corneal endothelial cells comprising float culturing in a medium comprising growth factor the human corneal endothelial precursor cells according to any of claims 1 to 5 or human corneal endothelial precursor cells prepared by the method according to any of claims 6 to 8.

19. A method of preparation of cellular aggregate derived from human corneal endothelial cells, comprising culturing human corneal endothelial cells in a medium comprising growth factor and glucose under human serum, animal serum, or no serum conditions; and then float culturing the cells obtained in a medium comprising growth factor.

20. The method of preparation according to claim 19, wherein human corneal endothelial cells are cultured in a primary culture and subcultured for 2 to 10 successive generations.

21. The method of preparation according to claims 19 or 20, wherein the culturing of the human corneal endothelial cells is conducted under conditions of 37°C and 5 to 10 percent CO₂.

22. The method of preparation according to any of claims 19 to 21, wherein the concentration of glucose in the medium comprising glucose is 2.0 g/L or lower.

23. The method of preparation according to any of claims 19 to 22, wherein the growth factor is one or more members selected from the group consisting of B cell growth factor (BCGF), epidermal growth factor (EGF), recombinant EGF (rEGF), and fibroblast growth factor (FGF).

24. The method of preparation according to any of claims 18 to 23, wherein the growth factor is one or more members selected from the group consisting of B27, epidermal growth factor (EGF), and basic fibroblast growth factor (bFGF).

25. A method of preparation of cellular aggregate derived from human corneal endothelial cells, comprising obtaining single cells by lysis of corneal endothelial cells with collagenase and then float culturing the cells obtained in a medium comprising growth factor.

26. The method of preparation according to claim 25, wherein the growth factor is one or more members selected from the group consisting of B27, epidermal growth factor (EGF), and basic fibroblast growth factor (bFGF).

27. The method of preparation according to any of claims 18 to 26, wherein the cellular aggregate has a diameter falling within a range of 30 to 500 micrometers.

28. A human corneal endothelium-like sheet, comprised of human corneal endothelium-like cells derived from the cellular aggregate according to any of claims 8 to 17 or a cellular aggregate prepared by the method according to any of claims 18 to 27, wherein the endothelium-like cells exhibit a polygonal form.

29. The sheet according to claim 28, wherein the polygonal form is a hexagon.

30. The sheet according to claim 28 or 29, wherein the mean cell density is 2,000 cells/mm² or greater.

31. The sheet according to any of claims 28 to 30, having transport activity.

32. The sheet according to claims 31, wherein the transport activity is equivalent to the transport activity of a normal human corneal endothelial layer.

33. The sheet according to any of claims 28 to 32, wherein the human corneal endothelium-like sheet is in the form adhered to a biodegradable support.

34. The sheet according to claim 33, wherein the support is one or more member selected from the group consisting of amniotic membrane, collagen membrane, cellulose membrane, and gelatin film.

35. A method of transplantation of human corneal endothelial precursor cells according to any of claims 1 to 5 or human corneal endothelial precursor cells prepared by the method according to any of claims 6 to 8 into the anterior chamber, comprising inserting a tube into the parenchyma of cornea, introducing the precursor cells into the anterior chamber through the inserted tube, and causing the human corneal endothelial precursor cells that have been introduced to adhere to Descemet's membrane.

36. A method of transplantation into the anterior chamber the cellular aggregate according to any of claims 9 to 17 or the cellular aggregate prepared by the method according to any of claims 18 to 27, comprising inserting a tube into the parenchyma of cornea, introducing the cellular aggregate into the anterior chamber through the inserted tube, and causing the cellular aggregate that has been introduced to adhere to Descemet's membrane.

37. The method according to claim 35 or 36, wherein the quantity of human corneal endothelial precursor cells introduced during single transplantation falls within a range of from 5,000 to 50,000 cells, or the quantity of cellular aggregate introduced falls within a range of 30 to 200 cellular aggregates.

38. The method according to any of claims 35 to 37, wherein the human corneal endothelial precursor cells or cellular aggregate is transplanted into the anterior chamber in the form of a mixture with a biodegradable support material.

39. The method according to claim 38, wherein the support material is a collagen sponge or gelatin microparticles.

40. A method of transplantation of the human corneal endothelium-like sheet according to any of claims 28 to 34 into the anterior chamber, comprising inserting a tube into the parenchyma of cornea, introducing the human corneal endothelium-like sheet into the anterior chamber through the tube that has been inserted, and causing the corneal endothelium-like sheet that has been introduced to adhere to Descemet's membrane.

41. The method according to any of claims 35 to 40, wherein the tube employed to introduce the human corneal endothelium precursor cells, cellular aggregate, or human corneal endothelium-like sheet is inserted into the parenchyma of cornea to a depth exceeding the thickness of the parenchyma of cornea.

42. The method according to any of claims 35 to 41, wherein the introduction of the human corneal endothelium precursor cells, cellular aggregate, or human corneal endothelium-like sheet into the anterior chamber is conducted after air is introduced into the anterior chamber, and a downward-facing position is assumed.

43. The method according to claim 42 wherein once the human corneal endothelium precursor cells, cellular aggregate, or human corneal endothelium-like sheet has been introduced into the anterior chamber, the downward-facing state is maintained for a prescribed period.

44. The method according to any of claims 35 to 43 wherein a solution containing human corneal endothelium precursor cells or cellular aggregate, or a corneal endothelium-like sheet, is introduced into the anterior chamber with an injector.

45. The method according to any of claims 35 to 44, being used to treat a disorder that causes corneal edema by reducing the number of corneal endothelial cells.

46. The method of claim 45, wherein the disorder is vesicular keratopathy, congenital hereditary endothelial corneal dystrophy, Fuchs' endothelial corneal dystrophy, guttate cornea, posterior polymorphic corneal dystrophy, iridocorneal endothelial syndrome, or a failed corneal transplant.

47. The method of claim 46, wherein the vesicular keratopathy is vesicular keratopathy following eye surgery, a laser iridectomy, uveitis, or an external injury.
